Europäisches Patentamt

European Patent Office

Office européen des brevets

(19)

(11) Publication number: **0 075 951**
A1

# (12) EUROPEAN PATENT APPLICATION

(21) Application number: 82109020.6

(22) Date of filing: 29.09.82

(51) Int. Cl.³: **C 07 C 29/10**
C 07 C 29/60, C 07 C 31/20
C 07 C 31/08

(30) Priority: 30.09.81 US 307210

(43) Date of publication of application:
06.04.83 Bulletin 83/14

(84) Designated Contracting States:
BE DE FR GB IT NL SE

(71) Applicant: UNION CARBIDE CORPORATION
Old Ridgebury Road
Danbury Connecticut 06817(US)

(72) Inventor: Bartley, William J.
500 Sheridan Circle
Charleston 25314 West Virginia(US)

(72) Inventor: Henry, Joseph Peter
453 Forest Circle
South Charleston 25303 West Virginia(US)

(74) Representative: Wuesthoff, Franz, Dr.-Ing. et al,
Patentanwälte Wuesthoff -v. Pechmann-Behrens-Goetz
Schweigerstrasse 2
D-8000 München 90(DE)

(54) Production of monoethylene glycol and ethanol from hydrogenolysis of polyalkylene glycols.

(57) This invention relates to a process for cleaving a polyalkylene glycol, e.g., diethylene glycol, containing at least one ether group therein at a carbon-to-oxygen covalent bond by heating the polyalkylene glycol with molecular hydrogen in the presence of a hydrogenation catalyst, e.g., copper chromite, to produce both monoethylene glycol and ethanol.

EP 0 075 951 A1

- 1 -

## PRODUCTION OF MONOETHYLENE GLYCOL AND ETHANOL FROM HYDROGENOLYSIS OF POLYALKYLENE GLYCOLS

### Brief Summary of the Invention

#### Technical Field

This invention is directed to the production of monoethylene glycol and ethanol by the hydrogenolysis of a polyalkylene glycol, e.g., diethylene glycol. The polyalkylene glycol is cleaved at a carbon-to-oxygen covalent bond by heating with molecular hydrogen in the presence of a hydrogenation catalyst, e.g., copper chromite, to produce both monoethylene glycol and ethanol.

#### Background Art

It is known that monoethylene glycol is commercially produced by the hydrolysis of ethylene oxide which in turn is generated by the oxidation of ethylene, typically by the reaction of ethylene and oxygen over a silver containing catalyst. Other processes (noncommercial) for making monoethylene glycol involve reactions of carbon monoxide with hydrogen or formaldehyde, typically in the presence of a certain precious metal catalyst. Major by-products of the commercial oxide/glycol process are diethylene glycol and higher polyalkylene glycols in amounts up to 10 percent by weight of the total product mixture. However, diethylene glycol may or may not be a commercially desirable by-product. For instance, considerably more diethylene glycol by-product may be produced from the commercial oxide/glycol process than can be utilized profitably. In those instances where the

- 2 -

production of diethylene glycol is commercially undesirable, any process capable of efficiently converting this by-product to monoethylene glycol, ethanol or other valuable products would be highly desirable.

It has been found as a result of the present invention that polyalkylene glycols, e.g., diethylene glycol, can be selectively cleaved at a carbon-to-oxygen covalent bond by heating the polyalkylene glycol with molecular hydrogen in the presence of a hydrogenation catalyst to efficiently produce at least one of monoethylene glycol and ethanol.

The following prior art references describe processes involving hydrogenolysis or cleaving with molecular hydrogen in the presence of a hydrogenation catalyst:

Japanese Patent Publication No. 25,246/74 describes selectively cleaving polyalkylene glycols, e.g., diethylene glycol, and polyalkylene glycol monoalkyl ethers at a carbon-to- carbon covalent bond by heating with molecular hydrogen in the presence of a nickel or palladium catalyst to give alkylene glycol monoalkyl ethers, polyalkylene glycol monoalkyl ethers, alkylene glycol dialkyl ethers and polyalkylene glycol dialkyl ethers. Lower polyalkylene glycols, monoethylene glycol, dimethyl ether and methane are described as being coproduced by this cleaving process. However, this patent does not disclose or teach the coproduction of ethanol resulting from the hydrogenolysis of a polyalkylene glycol.

German Patent 2,900,279 describes selectively cleaving polyalkylene glycols, e.g.,

diethylene glycol, or monoethylene glycol monoalkyl or monoaryl ethers including their homologs at a carbon-to-carbon covalent bond with molecular hydrogen in the presence of a catalyst consisting of a support material, onto which at least one of the elements palladium, platinum, ruthenium, rhodium or iridium are applied in the form of a compound to give dimethyl ethers, methylalkyl ethers or methylaryl ethers. When a polyalkylene glycol is used as the starting reactant, its terminal hydroxymethyl groups are cleaved to produce the dimethyl ether of a glycol. Monoethylene glycol and ethanol are not produced from the hydrogenolysis of a polyalkylene glycol according to the teachings of this patent.

French Patent 2,447,363 describes selectively cleaving polyalkylene glycol monoalkyl ethers at a carbon-to-oxygen covalent bond with molecular hydrogen in the presence of a nickel and barium catalyst to give alkylene glycol monoalkyl ethers and polyalkylene glycol monoalkyl ethers having one less ethylene group, e.g., diethylene glycol monoethyl ether is cleaved to give monoethylene glycol monoethyl ether. When hydrogenolysis of polyalkylene glycol monoalkyl ethers is not complete, small quantities of ethanol may be detected in the reaction mixture. This patent does not describe the hydrogenolysis of a polyalkylene glycol to produce both monoethylene glycol and ethanol.

U.S. Patent 3,833,634 describes a process for the preparation of polyfunctional oxygen-containing compounds such as ethylene glycol and/or derivatives thereof by reacting an oxide of carbon with hydrogen using a rhodium complex catalyst in combination with carbon monoxide at elevated temperature and pressure. Example 23 describes the production of ethylene glycol by hydrogenolysis of open chain glycol ethers, e.g., tetraethylene glycol dimethyl ether.

Japanese Public Disclosure No. 109,908/79 describes selectively cleaving an alkylene oxide, e.g., ethylene oxide, with molecular hydrogen in the presence of a hydrogenation catalyst and solvent having low polarity. The reaction is carried out in the liquid phase to give alkylene glycol alkyl ethers. Ethanol is produced as a by-product.

U.S. Patent 1,953,548 describes a process for producing n-propyl alcohol by passing propylene oxide vapor over a contact catalyst containing alumina at elevated temperature and immediately thereafter, in the presence of hydrogen, over a hydrogenation catalyst containing reduced nickel.

U.S. Patent 3,975,449 describes a hydrogenolysis process for producing a primary diol and/or triol from an epoxide by contacting the epoxide with molecular hydrogen and a solid catalyst comprising nickel or cobalt at elevated temperature and pressure.

Davidova and Kraus, _Journal of Catalysis_, _61_, 1-6 (1980) describe cleaving of 1,2-epoxybutane with molecular hydrogen in the presence of a supported platinum catalyst at elevated temperature and atmospheric pressure to give oxygenated products, e.g., 2-butanone, 1-butanol and 2-butanol.

German Patent 2,434,057 describes a hydrogenolysis method for producing glycol dimethyl ethers by cleaving the formals of the corresponding glycol monomethyl ethers with molecular hydrogen in the presence of catalysts composed of oxide mixtures of silicon and aluminum and/or rare earths and additionally containing the metals nickel, cobalt or copper.

German Patent 2,716,690 describes a hydrogenolysis method for producing glycol dimethyl ethers by cleaving the formals of the corresponding glycol monomethyl ethers with molecular hydrogen in the presence of catalysts composed of oxide mixtures of silicon and aluminum and/or rare earths as well as metallic nickel and/or cobalt and/or copper, characterized by the fact that the catalysts additionally contain metallic palladium and/or rhodium and/or platinum as the promoter.

However, none of these references and no prior art is currently known to us which discloses or teaches a process for cleaving a polyalkylene glycol at a carbon-to-oxygen covalent bond by heating with molecular hydrogen in the presence of a hydrogenation catalyst to produce at least one of monoethylene glycol and ethanol.

Disclosure of Invention

The present invention provides a process for cleaving a polyalkylene glycol, e.g., diethylene glycol, containing at least one ether group therein at a carbon-to-oxygen covalent bond by heating the polyalkylene glycol with molecular hydrogen in the presence of a hydrogenation catalyst, e.g., copper chromite, to produce at least one of monethylene glycol and ethanol.

In copending U.S. Patent Application Serial No. (D-13,340) filed on an even date herewith, there is described the manufacture of monoethylene glycol monomethyl ether, monoethylene glycol and ethanol by the hydrogenolysis of a polyalkylene glycol, e.g., -diethylene glycol. The polyalkylene glycol is selectivity cleaved at a carbon-to-oxygen covalent bond and independently at a carbon-to-carbon covalent bond by heating with molecular hydrogen in the presence of a hydrogenation catalyst comprising nickel to produce at least one of monoethylene glycol monomethyl ether, monoethylene glycol and ethanol. Monoethylene glycol monomethyl ether can be produced in predominant amounts by employing the process disclosed in this copending application, i.e., the selectivity of monoethylene glycol monomethyl ether may approach about 95 molar percent.

It has been surprisingly found that when the hydrogenation catalyst of the present invention is copper chromite, monoethylene glycol and ethanol are produced in selectivites which may approach or exceed 95 molar percent. Accordingly, it is readily apparent that either monoethylene glycol-ethanol or monoethylene glycol monomethyl ether may be selectively produced by similar hydrogenolysis processes utilizing either a hydrogenation catalyst such as copper chromite or a catalyst containing nickel respectively. The selectivities of both monoethylene glycol-ethanol and monoethylene glycol monomethyl ether may approach 95 molar percent in each of these inventions.

Copending U.S. Patent Application Serial No. (D-13,341), filed on an even date herewith, describes a process for selectively cleaving a polyalkylene glycol, e.g., diethylene glycol, at a carbon-to-oxygen covalent bond and independently at

- 7 -

a carbon-to-carbon covalent bond by heating with molecular hydrogen in the presence of a hydrogenation catalyst comprising ruthenium to produce at least one of monoethylene glycol monomethyl ether, monoethylene glycol and ethanol. The production rate of each of said monoethylene glycol monomethyl ether, monoethylene glycol and ethanol is at least about 10 moles/kilogram ruthenium/hour.

Copending U.S. Patent Application Serial No. (D-13,342), filed on an even date herewith, describes a process for selectively cleaving a polyalkylene glycol, e.g. diethylene glycol, at a carbon-to-oxygen covalent bond and independently at a carbon-to-carbon covalent bond by heating with molecular hydrogen in the presence of a hydrogenation catalyst comprising iridium to produce at least one of monoethylene glycol monoethyl ether, monoethylene glycol monomethyl ether, monoethylene glycol and ethanol. Monoethylene glycol monoethyl ether can be produced in significant amounts by employing the process disclosed in this copending application.

### Detailed Description

Hydrogenolysis of polyalkylene glycols may proceed according to different cleavage pathways. For example, polyalkylene glycols may be cleaved at either a carbon-to-oxygen covalent bond or a carbon-to-carbon covalent bond to give different products. The cleavage pathways are influenced or varied, to a certain extent, by choice of hydrogenation catalyst, concentration of active metal in the hydrogenation catalyst, pressure provided by molecular hydrogen and other factors. When a hydrogenation catalyst

0075951
13231

comprising copper chromite is used in the hydrogenolysis of a polyalkylene glycol, e.g., diethylene glycol, according to a preferred embodiment of the present invention, the cleavage pathway may be predominantly directed toward a carbon-to-oxygen covalent bond resulting in the production of monoethylene glycol and ethanol in predominant amounts. A lesser proportion of cleavage may be directed toward a carbon-to-carbon covalent bond resulting in the production of monoethylene glycol monomethyl ether in lesser amounts. As a preferred embodiment of the present invention, therefore, a hydrogenation catalyst containing copper chromite may be employed to obtain desirable amounts of monoethylene glycol and ethanol, i.e., monoethylene glycol and ethanol may be produced in selectivities which may approach or exceed 95 molar percent.

The preferred hydrogenation catalysts which can be used to catalyze the hydrogenolysis process of this invention are well known in the art and include compositions containing: copper, chromium, molybdenum, tungsten, iron, cobalt, nickel, ruthenium, rhodium, palladium, platinum, iridium, rhenium, tantalum, vanadium, niobium, zinc or combinations thereof. The preferred hydrogenation catalysts may also include, as an additional ingredient, a promoter or promoters for increasing the activity of the hydrogenation catalysts. Suitable catalyst promoters include barium, calcium, magnesium, strontium, zirconium, titanium, hafnium, potassium, lithium, sodium, rubidium, cesium, manganese, molybdenum, chromium, tungsten, zinc and the like. Certain metals such as molybdenum, chromium, tungsten and zinc may act as either a co-catalyst or promoter. Reference to the hydrogenation catalysts and promoters as containing the above-described metals is meant to include the metal in the compound form as well as the elemental form, for example, as a metal oxide or sulfide or as a metal hydride, or as the reduced elemental metal or as mixtures of the foregoing. The hydrogenation catalysts may be employed in the presence or absence of a catalyst support. Suitable catalyst supports include silica gel, aluminum oxide, magnesium oxide, titanium dioxide, magnesium aluminate, zirconium dioxide, silicon carbide, ceramic, pumice, carbon, diatomaceous earth, kieselguhr and the like. Illustrative of the preferred hydrogenation catalysts useful in the process of this invention include compositions containing: cuprous oxide and dichromium trioxide (copper chromite); cuprous oxide, dichromium trioxide and barium oxide; cuprous oxide on silica gel; cuprous molybdate and potassium monoxide; cuprous oxide and zinc oxide; cuprous oxide, zinc oxide and dichromium trioxide; zinc oxide and dichromium trioxide; cuprous oxide and dichromium trioxide on silica gel; cobaltous and/or cobaltic oxide; cobaltous and/or cobaltic oxide on kieselguhr; cobaltous and/or cobaltic oxide and zirconium dioxide on kieselguhr; cobalt molybdate on silica gel or aluminum oxide; ferric oxide on aluminum oxide; ferric oxide on silica gel; iridium on silica gel; molybdenum trioxide on aluminum oxide; molybdenum dioxide on aluminum oxide; nickel; nickel on silica gel; nickel on kieselguhr; palladium on carbon; palladium on aluminum oxide; palladium on silica gel; palladium and calcium oxide on silica gel; palladium and molybdenum oxide on silica gel; platinum on carbon; platinum on silica gel; platinum and calcium oxide on silica gel; rhenium on silica gel; rhodium on aluminum oxide; rhodium on silica gel; rhodium and iron on magnesium oxide; rhodium and calcium oxide on silica gel; ruthenium on magnesium oxide; tantalum oxide on aluminum oxide; tungsten trioxide on aluminum oxide; and cuprous oxide, dichromium trioxide and manganese oxide. The compositions containing oxides may be totally or partially reduced in situ during reactor heat-up. It is noted that hydrogenation catalysts containing iridium, ruthenium or nickel are also utilized in separate hydrogenolysis processes which are the subject matter of the copending U.S. patent applications discussed hereinabove. The most preferred hydrogenation catalysts of this invention include compositions containing copper, and chromium with or without barium, e.g., cuprous oxide, dichromium trioxide with or without barium oxide. Many of the above-described hydrogenation catalysts are commercially available and others are prepared by known methods detailed hereinbelow.

The hydrogenation catalysts useful in the process of this invention can be employed in any form. They may be present in the reaction mixture as finely divided powders, porous tablets, pellets, spheres, granules and the like. It is desirable that the hydrogenation catalysts possess a high surface area to provide preferred reaction rates. The preferred surface area ranges from about 0.5 $m^2/gm$ to about 300 $m^2/gm$ and even greater. Most preferably, the surface area of the hydrogenation catalysts ranges from about 10 $m^2$/gm to about 300 $m^2$/gm. The average pore diameter of the hydrogenation catalysts is not narrowly critical. Hydrogen diffusion appears to be rate limiting in catalysts having very small pores. It also appears that a very small average pore diameter and/or a large catalyst particle diameter are detrimental to high monoethylene glycol-ethanol selectivities and/or polyalkylene glycol conversion rates. In general, for obtaining desirable monoethylene glycol-ethanol selectivities, catalysts having an average pore diameter of at least about 150 Å (15 nm), preferably at least about 250 Å (25 nm), and a particle diameter of less than 10 millimeters, preferably 5 millimeters or less, are used in the hydrogenolysis process of this invention.

The quantity of the hydrogenation catalyst which is employed is not narrowly critical and can vary over a wide range. In general, the process of this invention is desirably conducted either continuously or batchwise in the presence of a catalytically effective quantity of the hydrogenation catalyst which gives a suitable and reasonable reaction rate, e.g., from about 1 to about 100 moles/kilogram catalyst/hour. The reaction proceeds when one employs as little as about0.01 weight percent, or even a lesser amount of catalyst based on the total quantity of the reaction mixture under batch conditions. The upper concentration of the hydrogenation catalyst can be quite high, e.g., about 50 weight percent of catalyst based on the total quantity of reaction mixture. Higher concentrations may be used if desired. However, the upper concentration appears to be dictated by economics in terms of the cost of certain hydrogenation catalysts to achieve the given reaction and by ease of handling of the reaction mixture during the course of the reaction. Depending on various factors, such as the total operative pressure of the system, the operative temperature and other considerations, a concentration of between about 0.1 and about 30 weight percent hydrogenation catalyst based on the total quantity of the reaction mixture is generally suitable in the practice of this invention.

The preferred polyalkylene glycols which can be used in the practice of the process of this invention are well known in the art and include: diethylene glycol, triethylene glycol, tetraethylene glycol and the like. In the most preferred embodiment of this invention, diethylene glycol formed as a by-product of the commercial oxide/glycol process described hereinabove, is cleaved at a carbon-to-oxygen covalent bond by heating with molecular hydrogen in the presence of a hydrogenation catalyst to produce at least one of monoethylene glycol and ethanol. By utilizing the preferred catalysts and reaction conditions of the present invention, higher polyalkylene glycols such as triethylene glycol and tetraethylene glycol may be selectively cleaved to give at least one of monoethylene glycol and ethanol. These polyalkylene glycols are prepared by methods well known in the art.

The relative amounts of polyalkylene glycol and molecular hydrogen which are initially present in the reaction mixture can be varied over a wide range. In general, the molar ratio of molecular hydrogen to polyalkylene glycol is in the range of between about 1:20 and about 2,000:1, preferably between about 1:2 and about 500:1 and most preferably, between 1:1 and about 200:1. It is to be understood, however, that molar ratios outside the stated broad range may be employed. However, very high molecular hydrogen-to-polyalkylene glycol molar ratios are economically unattractive, and very low molecular hydrogen-to-polyalkylene glycol molar ratios will result generally in low conversions and low product selectivities. When operating in the continuous mode, molecular hydrogen and polyalkylene glycol may be fed either concurrently or countercurrently over the hydrogenation catalyst using either upward, downward or horizontal flow, preferably concurrently downward flow. The gas hourly space velocity (GHSV) of molecular hydrogen and the liquid hourly space velocity (LHSV) of polyalkylene glycol may be varied over a wide range utilizing the continuous process. The gas hourly space velocity of molecular hydrogen may range from about 100 $hr^{-1}$ or less to greater than 100,000 $hr^{-1}$, preferably from about 1,000 $hr^{-1}$ to about 100,000 $hr^{-1}$, and most preferably from about 2,000 $hr^{-1}$ to about 75,000 $hr^{-1}$. The liquid hourly space velocity of polyalkylene glycol may range from about .01 $hr^{-1}$ or less to about 100 $hr^{-1}$ or greater, preferably from about 0.1 $hr^{-1}$ to about 25 $hr^{-1}$, and most preferably from about 0.25 $hr^{-1}$ to about 10 $hr^{-1}$. The higher GHSV and LHSV values generally tend to result in uneconomically low conversion of polyalkylene glycol, and the lower GHSV and LHSV values generally tend to decrease the selectivity of monoethylene glycol and ethanol. The intended purpose is to provide a sufficient quantity of polyalkylene glycol and molecular hydrogen in the reaction mixture to effect the desired production of monoethylene glycol and ethanol.

The process of this invention can be effected over a wide temperature range. In general, the process is conducted at a temperature of between about 150°C and about 350°C. Lower temperatures generally give improved product selectivity, but often at economically unfavorable rates. Higher temperatures generally increase total productivity but tend to increase by-product formation. Operating the process at temperatures lower than 150°C will not produce the desired products at optimum rate so that the reaction will typically be operated over an extended period of time and/or excessive quantities of catalyst must be employed in order to obtain the desired production of reaction products at reasonable rates. When operating the process at temperatures higher than 350°C there is a tendency for the organic materials contained in the reaction mixture to decompose. In most cases when operating at the lower end of the temperature range, it is desirable to utilize pressures in the higher end of the pressure range. The preferred temperature range is between about 200°C and 300°C, while the most preferred temperature range is between about 225°C and 265°C. However, there are occasions when a preferred temperature range may include any of the more desirable ranges as well as the broadest range such that the process may be operated at a temperature of between 175°C and 300°C as well as between about 150°C and 325°C.

The process of the present invention is effected under atmospheric or superatmospheric pressure conditions. In a preferred embodiment of the present invention, the pressure is produced almost exclusively by the molecular hydrogen provided to the reaction. However, suitable inert gaseous diluents may also be employed if desired. Actual operating pressures (initial pressures under batch operating conditions) of between about 690 bar atmospheric and about 10,000 psig represent an operative limit for producing the monoethylene glycol and ethanol products. The preferred actual operating pressure conditions for production of 34.5 bar 276 bar monoethylene glycol and ethanol ranges from about 500 psig to about 4000 psig and, most preferably, 69 bar 207 bar from about 1000 psig to about 3000 psig. When operating the process at lower pressures, the rate of reaction becomes slower and therefore the reaction period must be extended until the desired amount of reaction products are produced. When operating the process at higher pressures, the rate of production of the desired products will be increased. Higher pressures generally increase the selectivity of monoethylene glycol and ethanol but very high pressures increase the secondary hydrogenolysis of monoethylene glycol and ethanol. Lower pressures increase the selectivity of ethylene glycol monoalkyl ethers, increase the formation of by-products such as 2-oxodioxane and decrease the selectivity of monoethylene glycol and ethanol.

The process of this invention is effected for a period of time sufficient to produce the desired monoethylene glycol and ethanol products. In general, the reaction time can vary from a fraction of a second to several hours i.e., from a 0.1 second and shorter to approximately 10 hours, and longer. If more sluggish reaction conditions are selected, then the reaction time will have to be extended until the desired products are produced. It is readily appreciated that the required residence period, i.e., reaction time, will be influenced by reaction temperature, concentration and choice of hydrogenation catalyst, total pressure, concentration (molar ratio) of polyalkylene glycol and molecular hydrogen and other factors. When operating the hydrogenolysis process of this invention under continuous conditions, the reaction time or residence period may generally range from a fraction of a second to several minutes or longer whereas the residence period under batch conditions may generally range from a few minutes to several hours. The synthesis of the desired products from the hydrogenolysis of a polyalkylene glycol is suitably conducted under operative conditions which give reasonable reaction rates and/or conversions.

The process of this invention can be carried out in a batch, semi-continuous or continuous manner in either a liquid phase, vapor phase or a combination liquid-vapor phase. The reaction may be conducted in fixed-bed, slurry-phase, trickle-bed or fluidized-bed reactors or the like using a single reaction zone or in a plurality of reaction zones, in series or in parallel. The reaction may be conducted intermittently or continuously in an elongated tubular zone or a series of such zones. The material of construction of the equipment should be such so as to be inert during the reaction. The equipment should also be able to withstand the reaction temperatures and pressures. The reaction zone can be fitted with internal and/or external heat exchangers to control undue temperature fluctuations caused by the moderate exothermic nature of the reaction. In a preferred embodiment of the present invention, agitation means to ensure complete mixing of the reaction mixture should be used in reaction systems not employing a fixed-bed catalyst. Mixing induced by magnetic stirrer, vibration, shaker, rotating, sparging with gas, oscillation, etc. are all illustrative of the types of agitation means which are contemplated herein. Such agitation means are available and well known to the art.

The polyakylene glycol, molecular hydrogen and hydrogenation catalyst ingredients may be initially introduced into the reaction zone batchwise. Alternatively, the polyalkylene glycol, molecular hydrogen and hydrogenation catalyst ingredients may be introduced into the reaction zone continuously or intermittently during the course of the synthesis reaction. Means to introduce the ingredients into the reaction zone during the course of the reaction and/or means to adjust the ingredients in the reaction zone during the reaction, either intermittently or continuously, can be conveniently utilized in the process to maintain the desired molar ratios of ingredients and to maintain the pressure exerted by hydrogen.

The operative conditions of the present process may be adjusted to optimize the selectivities and conversion rates of the desired products and/or the economics of the process. For example, it may generally be preferred to operate at relatively low conversions which tends to increase selectivities of monoethylene glycol and ethanol due to reduced by-product formation. Lower conversions also tend to increase the monoethylene glycol/ethanol molar ratio by reducing secondary hydrogenolysis of monethylene glycol. Recovery of the desired monethylene glycol and ethanol can be achieved by methods well known in the art, such as by distillation, fractionation, extraction, and the like. Typically, in carrying out the process, the product contained in the reaction mixture would be withdrawn from the reaction zone and distilled to recover the desired products. For reactors not employing a fixed-bed catalyst, a fraction comprising the hydrogenation catalyst and by-products can be removed for recovery or regeneration of the catalyst. Fresh hydrogenation catalyst can be intermittently added to the reaction stream or it can be added directly to the reaction zone, to replenish any catalyst which is lost in the process.

Although this invention has been described with respect to a number of details, it is not intended that this invention should be limited thereby. The examples which follow are intended solely to illustrate the embodiments of this invention which to date have been determined and are not intended in any way to limit the scope and the intent of this invention.

As used in the Examples appearing hereinafter, the following designations, terms and abbreviations have the indicated meanings:

psig:           pounds per square inch gauge pressure.
rpm:            revolutions per minute.
kg:             kilogram
lbs.:           pounds    (0,45 kg)
cu.:            cubic
ft.:            foot      (0,3 m)
cat.:           catalyst
hr.:            hour
cc:             cubic centimeter
mm:             millimeter
m$^2$           square meter
gm:             gram
cm$^2$          square centimeter
conv.:          conversion
DEG:            diethylene glycol
GHSV:           gas hourly space velocity
LHSV:           liquid hourly space velocity
hr':            reciprocal hour unit for GHSV and LHSV
% or percent:   percent by weight unless otherwise
                specified.
ratios:         are on a weight basis unless otherwise
                specified.
temperatures:   are given in °C unless otherwise
                specified.
ND:             not detected
selectivity:    calculated according to the formula,

$$\% \text{ Selectivity } i = \frac{r_i \times 100}{r_{DEG} \text{ conv.}}$$

where i is the individual component, i.e., monethylene glycol, ethanol or by-products, and r is the rate in moles/kg cat/hr.

The catalysts used in the Examples are identified as follows:

Catalyst I. A composition containing 40 percent by weight cuprous oxide, 42 percent by weight dichromium trioxide and 5 percent by weight barium oxide; having a surface area of 80-130 m$^2$/gm, a bulk density of 22-25 lbs./cu. ft. and a particle size of less than 325 mesh; and sold by Calsicat as Calsicat E-101P.

352 - 400 kg/m$^3$

44 µm

Catalyst II. A composition containing 45 percent by weight cuprous oxide, 45 percent by weight dichromium trioxide and 6 percent by weight barium oxide; having a surface area of 30-60 $m^2$/gm, a bulk density of 25-28 lbs./cu. ft. [400 - 448 $kg/m^3$] and a particle size of less than 325 mesh [44 $\mu m$]; and sold by Calsicat as Calsicat E-102P.

Catalyst III. A composition containing 47 percent by weight cuprous oxide and 47 percent by weight dichromium trioxide; having a surface area of 90-100 $m^2$/gm, a bulk density of 90-95 lbs./cu.ft. [1440 - 1520 $kg/m^3$], a crush strength of 20 lbs. [9 $kg$], a particle size of 1/8 [3,2 x] inch by 1/8 inch [3,2 $mm$] and a pore volume of 0.20 cc/gm; and sold by Calsicat as Calsicat E-103TR.

Catalyst IV. A composition containing 43 percent by weight cuprous oxide, 43 percent by weight dichromium trioxide and 8 percent by weight barium oxide; having a surface area of 85 $m^2$/gm, a bulk density of 90-95 lbs./cu.ft. [1440 - 1520 $kg/m^3$], a crush strength of 12 lbs. [5,3 $kg$], a particle size of 1/8 inch [3,2 x] by 1/8 inch [3,2 $mm$] and a pore volume of 0.20 cc/gm; and sold by Calsicat as Calsicat E-106TR.

Catalyst V. A composition containing 78 percent by weight cuprous oxide and 20 percent by weight dichromium trioxide; having a surface area of 6 $m^2$/gm, a bulk density of 137 lbs./cu. ft. [2192 $kg/m^3$], a particle size of 1/8 inch [3,2 x] by 1/8 inch [3,2 $mm$] and a pore volume of 0.10 cc/gm; and sold by Harshaw as Harshaw Cu-0203T.

Catalyst VI. A composition containing 35 percent by weight cuprous oxide, 38 percent by weight dichromium trioxide and 10 percent by weight barium oxide; having a surface area of 14-15 $m^2$/gm, a bulk density of 54 lbs/cu. ft. [864 $kg/m^3$], a crush

— 21 —

strength of 8-12 lbs., a particle size of 1/8 inch *3,6 – 5,4 kg* *3,2 x* by 1/8 inch and a pore volume of 0.47 cc/gm; and sold *3,2 mm* by Harshaw as Harshaw Cu-0402T.

Catalyst VII. A composition containing 33 percent by weight cuprous oxide, 38 percent by weight dichromium trioxide and 9 percent by weight barium oxide; having a surface area of 35 $m^2$/gm, a bulk density of 105 lbs./cu. ft., a crush strength *1680 kg/m3* of 18 lbs., a particle size of 1/8 inch by 1/8 inch *8,1 kg* *3,2 x 3,2 mm* and a pore volume of 0.10 cc/gm; and sold by Harshaw as Harshaw Cu-1107T.

Catalyst VIII. A composition containing 39 percent by weight cuprous oxide, 43.5 percent by weight dichromium trioxide and 10 percent by weight barium oxide; having a surface area of 50 $m^2$/gm, a bulk density of 30 lbs./cu.ft., the particle size is *480 kg/m3* powder and a pore volume of 0.76 cc/gm; and sold by Harshaw as Harshaw Cu-1106P.

Catalyst IX. A composition containing 41 percent by weight cuprous oxide, 45 percent by weight dichromium trioxide and 10 percent by weight barium oxide; having a surface area of 50 $m^2$/gm, a bulk density of 30 lbs./cu.ft. and the particle size *480 kg/m3* is powder; and sold by Harshaw as Harshaw Cu-1114P.

Catalyst X. A composition containing 34 percent by weight cuprous oxide, 37 percent by weight dichromium trioxide and 8 percent by weight barium oxide; having a surface area of 24 $m^2$/gm, a bulk density of 118 lbs./cu.ft., a crush strength of *1888 kg/m3* 23 lbs., a particle size of 1/8 inch by 1/8 inch and *10,35 kg* *3,2 x 3,2 mm* a pore volume of 0.10 cc/gm; and sold by Harshaw as Harshaw Cu-1117T.

- 22 -

Catalyst XI. A composition containing 38 percent by weight cuprous oxide, 38 percent by weight dichromium trioxide and 9 percent by weight barium oxide; having a surface area of 30 m²/gm, a bulk density of 110 lbs./cu.ft., a crush strength of 16 lbs., a particle size of 1/8 inch by 1/8 inch and a pore volume of 0.16 cc/gm; and sold by Harshaw as Harshaw Cu-1132T.

Catalyst XII. A composition containing 42 percent by weight cuprous oxide and 38 percent by weight dichromium trioxide; having a surface area of 30 m²/gm, a bulk density of 93 lbs./cu.ft., a crush strength of 15 lbs., a particle size of 1/8 inch by 1/8 inch and a pore volume of 0.21 cc/gm; and sold by Harshaw as Harshaw Cu-1808T.

Catalyst XIII. A composition containing 50 percent by weight cuprous oxide and 37 percent by weight dichromium trioxide; having a particle size of 3/16 inch; and sold by Girdler as Girdler G-13.

Catalyst XIV. A composition containing 45 percent by weight cuprous oxide, 45 percent by weight dichromium trioxide and 4 percent by weight manganese oxide; having a bulk density of 81 lbs./cu.ft., a crush strength of 180 kg/cm² and a particle size of 5 mm; and sold by Nikki as Nikki N-201.

Catalyst XV. A composition containing 43 percent by weight cuprous oxide and 39 percent by weight dichromium trioxide; having a surface are of 49 m²/gm, a bulk density of 93 lbs./cu.ft., a crush strength of 14 lbs., a particle size of 1/8 inch and by 1/8 inch and a pore volume of 0.21 cc/gm; and sold by Harshaw as Harshaw Cu-1809T.

Catalyst XVI. ·A composition containing 40 percent by weight cuprous oxide and 60 percent by weight dichromium trioxide; having a surface area of 30 m$^2$/gm, a bulk density of 73 lbs./cu.ft., the particle size is powder and a pore volume of 0.69 cc/gm; and sold by Harshaw as Harshaw Cu-1402P. *1168 kg/m$^3$*

Catalyst XVII. A composition containing 51 percent by weight cuprous oxide and 46 percent by weight dichromium trioxide; having a surface area of 30 m$^2$/gm, a bulk density of 41 lbs./cu.ft., the particle size was powder and a pore volume of 0.76 cc/gm; and sold by Harshaw as Harshaw Cu-1800P. *656 kg/m$^3$*

Catalyst XVIII. A composition containing 39 percent by weight cuprous oxide and 50 percent by weight dichromium trioxide; having a surface area of 15 m$^2$/gm, a bulk density of 100 lbs./cu.ft., a crush strength of 9 lbs., a particle size of 1/8 inch by 1/8 inch and a pore volume of .18 cc/gm; and sold by Harshaw as Harshaw Cu-1422T. *1600 kg/m$^3$*  *4 kg* *x 3,2 mm* *3,2*

Catalyst XIX. A composition containing 7.5 percent by weight cuprous oxide on silica; having a surface area of 4 m$^2$/gm, a bulk density of 30 lbs./cu.ft., a particle size of 4-10 mesh and a pore volume of .61 cc/gm; and sold by Harshaw as Harshaw Cu-2501. *480 kg/m$^3$*  *2 - 4,76 μm*

Catalyst XX. A composition containing 33 percent by weight cuprous oxide and 65 percent by weight zinc oxide; having a particle size of 3/16 inch; and sold by Girdler as Girdler G-66B. *4,8 mm*

Catalyst XXI. A composition containing cuprous oxide, zinc oxide and dichromium trioxide; having a particle size of 1/8 inch by 1/4 inch; and sold by Catalysts & Chemicals, Inc. as Catalysts & Chemicals, Inc. C-79-2. *3,2 x 6,75 mm*

- 24 -

Catalyst XXII. A composition containing zinc oxide and dichromium trioxide; having a bulk density of 85 lbs./cu.ft. and a particle size of 1/8 inch by 1/4 inch; and sold by Catalysts & Chemicals, Inc. as Catalysts & Chemicals, Inc. C-70-2.

Catalyst XXIII. A composition containing 15 percent by weight cuprous oxide and 31 percent by weight zinc oxide; having a particle size of 1/8 inch by 1/4 inch; and sold by Catalysts & Chemicals, Inc. as Catalysts & Chemicals, Inc. C-18-1.

Catalyst XXIV. A composition containing 11 percent by weight cuprous oxide and 0.55 percent by weight dichromium trioxide on silica gel; having a particle size of 1/8 inch; and sold by Girdler as Girdler G-1576.

Catalyst XXV. A composition containing 22 percent by weight cobaltous-cobaltic oxide on silica; having a surface area of 110 $m^2$/gm, a particle size of 1/4 inch and a pore volume of 0.21 cc/gm; and sold by Girdler as Girdler T-1640.

Catalyst XXVI. A composition containing 35 percent by weight cobaltous oxide on kieselguhr; having a surface area of 130 $m^2$/gm, a bulk density of 71 lbs./cu.ft., a crush strength of 17.5 lbs., a particle size of 3/16 inch and a pore volume of 0.35 cc/gm; and sold by Harshaw as Harshaw Co-101.

Catalyst XXVII. A composition containing 52 percent by weight cobaltous oxide and 2.5 percent by weight zirconium dioxide on kieselguhr; having a particle size of 3/16 inch; and sold by Girdler as Girdler G-67.

Catalyst XXVIII. A composition containing 3 percent by weight cobaltous oxide and 15 percent by weight molybdenum trioxide on silica alumina; having a surface area of 180 m$^2$/gm, a bulk density of 60 lbs./cu.ft., a crush strength of 24 lbs., a particle size of 1/8 inch and a pore volume of 0.40 cc/gm; and sold by Harshaw as Harshaw CoMo-0402.

960 kg/m3    10,8 kg    3,2 mm

Catalyst XXIX. A composition containing cobaltous oxide and molybdenum trioxide on alumina; having a particle size of 1/8 inch; and sold by Girdler as Girdler G-39.

3,2 mm

Catalyst XXX. A composition containing 10 percent by weight molybdenum trioxide on aluminum oxide; having a surface area of 160 m$^2$/gm, a bulk density of 53 lbs./cu.ft., a crush strenght of 19 lbs., a particle size of 1/8 inch and a pore volume of 0.36 cc/gm; and sold by Harshaw as Harshaw Mo-1201.

848 kg/m3    8,6 kg    3,2 mm

Catalyst XXXI. A composition containing 10 percent by weight molybdenum trioxide on aluminum oxide; having a particle size of 1/8 inch by 1/4 inch; and sold by Houdry.

3,2 x 6,35 mm

Catalyst XXXII. A composition containing 10 percent by weight molybdenum trioxide on aluminum oxide; having a surface area of 64 m$^2$/gm, a bulk density of 61 lbs./cu.ft., a crush strenght of 10 lbs, a particle size of 1/8 inch and a pore volume of 0.40 cc/gm; and sold by Harshaw as Harshaw Mo-0502.

976 kg/m3    4,5 kg    3,2 mm

Catalyst XXXIII. A composition containing 5 percent by weight palladium on carbon; having a bulk density of 20 lbs./cu.ft. and the particle size is powder; and sold by Engelhard.

320 kg/m3

Catalyst XXXIV. A composition containing 5 percent by weight platinum on carbon; the particle size is powder; and sold by Matthey Bishop as Matthey Bishop BD-533.

Catalyst XXXV. A composition containing 5 percent by weight sulfided platinum on carbon; the particle size is powder; and sold by Engelhard as Engelhard 11-217.

Catalyst XXXVI. A composition containing 0.5 percent by weight rhodium on aluminum oxide; having a particle size of 1/8 inch; 3,2 mm and sold by Engelhard as Engelhard 10034.

Catalyst XXXVII. A composition containing 5 percent by weight ruthenium on carbon; the particle size is powder; and sold by Matthey Bishop.

Catalyst XXXVIII. A composition containing 10 percent by weight cobaltous oxide and 0.5 percent by weight calcium on silica gel; the silica gel is commercially available from Davison Specialty Chemical Company as Grade-59; and having a surface area of 275 $m^2$/gm, a bulk density of 25 lbs./cu. 400 kg/m$^3$ ft., the particle size is powder after grinding and a pore volume of 1.2 cc/gm.

Catalyst XXXIX. A composition containg 10 percent by weight nickel on silica gel; the silica gel is commercially available from Davison Specialty Chemical Company as Grade-59; and having a surface area of 275 $m^2$/gm, a bulk density of 25 lbs./cu. 400 kg/m$^3$ ft., the particle size is powder after grinding and a pore volume of 1.2 cc/gm.

Catalyst XL. A composition containing 5 percent by weight palladium on silica gel; the silica gel is commercially available from Davison Specialty Chemical Company as Grade-59; and having a surface area of 275 $m^2$/gm, a bulk density of 25 400 kg/m$^3$ lbs./cu. ft., the particle size is powder after grinding and a pore volume of 1.2 cc/gm.

Catalyst XLI. A composition containing 5 percent by weight palladium and 0.5 percent by weight calcium on silica gel; the silica gel is commercially available from Davison Specialty Chemical Company as Grade-59; and having a surface area of 275 $m^2$/gm, a bulk density of 25 lbs./cu. 400 kg/m³ ft., the particle size is powder after grinding and a pore volume of 1.2 cc/gm.

Catalyst XLII. A composition containing 5 percent by weight palladium and .2 percent by weight calcium on silica gel; the silica gel is commercially available from Davison Specialty Chemical Company as Grade-59; and having a surface area of 275 $m^2$/gm, a bulk density of 25 lbs./cu. 400 kg/m³ ft., the particle size is powder after grinding and a pore volume of 1.2 cc/gm.

Catalyst XLIII. A composition containing 5 percent by weight palladium and .1 percent by weight calcium on silica gel; the silica gel is commercially available from Davison Specialty Chemical Company as Grade-59; and having a surface area of 275 $m^2$/gm, a bulk density of 25 lbs./cu. 400 kg/m³ ft., the particle size is powder after grinding and a pore volume of 1.2 cc/gm.

Catalyst XLIV. A composition containing 5 percent by weight palladium and .02 percent by weight calcium on silica gel; the silica gel is commercially available from Davison Specialty Chemical Company as Grade-59; and having a surface area of 275 $m^2$/gm, a bulk density of 25 lbs./cu. 400 kg/m³ ft., the particle size is powder after grinding and a pore volume of 1.2 cc/gm.

Catalyst XLV. A composition containing 5 percent by weight palladium and 1 percent by weight calcium on silica gel; the silica gel is commercially available from Davison Specialty Chemical Company as Grade-59; and having a surface area of 275 $m^2$/gm, a bulk density of 25 lbs./cu. ft., [400 kg/m³] the particle size is powder after grinding and a pore volume of 1.2 cc/gm.

Catalyst XLVI. A composition containing 5 percent by weight palladium and 2 percent by weight molybdenum on silica gel; the silica gel is commercially available from Davison Specialty Chemical Company as Grade-59; and having a surface area of 275 $m^2$/gm, a bulk density of 25 lbs./cu. [400 kg/m³] ft., the particle size is powder after grinding and a pore volume of 1.2 cc/gm.

Catalyst XLVII. A composition containing 5 percent by weight platinum and .2 percent by weight calcium on silica gel; the silica gel is commercially available from Davison Specialty Chemical Company as Grade-59; and having a surface area of 275 $m^2$/gm, a bulk density of 25 lbs./cu. [400 kg/m³] ft., the particle size is powder after grinding and a pore volume of 1.2 cc/gm.

Catalyst XLVIII. A composition containing 5 percent by weight rhenium on silica gel; the silica gel is commercially available from Davison Specialty Chemical Company as Grade-59; and having a surface area of 275 $m^2$/gm, a bulk density of 25 [400 kg/m³] lbs./cu. ft., the particle size is powder after grinding and a pore volume of 1.2 cc/gm.

Catalyst XLIX. A composition containing 10 percent by weight rhodium on silica gel; the silica gel is commercially available from Davison Specialty Chemical Company as Grade-59; and having a surface area of 275 $m^2$/gm, a bulk density of 25 lbs./cu. [400 kg/m³]

ft., the particle size is powder after grinding and a pore volume of 1.2 cc/gm.

Catalyst L. A composition containing 2.5 percent by weight rhodium and 2 percent by weight calcium on silica gel; the silica gel is commercially available from Davison Specialty Chemical Company as Grade-59; and having a surface area of 275 $m^2$/gm, a bulk density of 25 lbs./cu. 400 kg/m³ ft., the particle size is powder after grinding and a pore volume of 1.2 cc/gm.

Catalyst LI. A composition containing 10 percent by weight ferric oxide on silica gel; the silica gel is commercially available from Davison Specialty Chemical Company as Grade-59; and having a surface area of 275 $m^2$/gm, a bulk density of 25 400 kg/m³ lbs./cu. ft., the particle size is powder after grinding and a pore volume of 1.2 cc/gm.

Catalyst LII. A composition containing .5 percent by weight palladium on aluminum oxide; the aluminum oxide (alpha alumina) is commercially available from Norton as LA-4102; and having a surface area of less than 1 $m^2$/gm, a particle size 3,2 mm of 3/16 inch and the pore volume is 0.35 cc/gm.

Catalyst LIII. A composition containing 5 percent by weight platinum on silica gel; the silica gel is commercially available from Davison Specialty Chemical Company as Grade-59; and having a surface area of 275 $m^2$/gm, a bulk density of 25 lbs./cu. 400 kg/m³ ft., the particle size is powder after grinding and a pore volume of 1.2 cc/gm.

Catalyst LIV. A composition containing 2.5 percent by weight rhodium and 0.5 percent by weight iron on magnesium oxide; and having a surface area of 125 $m^2$/gm, a bulk density of 44 lbs./cu. ft., a 704 kg/m³

- 30 -

particle size of 1/8 inch by 3/16 inch and the pore volume is 0.44 cc/gm.

3,2 mm     4,8 mm

Catalyst LV. A composition containing 20 percent by weight molybdenum dioxide on aluminum oxide; the aluminum oxide (alpha alumina) is commercially available from Norton as SA-5105; and having a surface area of less than 1 $m^2$/gm and the bulk density is 100 lbs./cu. ft.

1600 kg/m³

Catalyst LVI. A composition containing 20 percent by weight tantalum oxide on aluminum oxide; the aluminum oxide (alpha alumina) is commercially available from Norton as SA-5105; and having a surface area of less than 1 $m^2$/gm and the bulk density is 100 lbs./cu. ft.

1600 kg/m3

Catalyst LVII. A composition containing 20 percent by weight tungsten trioxide on aluminum oxide; the aluminum oxide (alpha alumina) is commercially available from Norton as SA-5105; and having a surface area of less than 1 $m^2$/gm and the bulk density is 100 lbs./cu. ft.

1600 kg/m³

Catalyst LVIII. A composition containing 95 percent by weight cuprous molybdate and 5 percent by weight potassium monoxide.

Commercially available catalysts, i.e., Catalysts I through XXXVII, were used in the Examples as supplied from the manufacturer. In the Examples conducted under batch conditions, the pelleted versions were first pulverized using a mortar and pestle until each catalyst could pass through a 200-mesh screen. In the Examples conducted under continuous conditions, the catalyst particles as received from the manufacturer were first broken and then sieved to 8-20 mesh (Examples 80 through 84 and 98 through 104) or 18-30 mesh

0,84 - 2,38 mm
0,59 - 1 mm

(Examples 85 through 97 and 105 through 110). Catalyst reduction, where necessary, was accomplished in situ during reactor heat-up. It should be noted that Girdler and Catalyst & Chemicals, Inc. have combined and are now doing business as United Catalysts, Inc.

Catalysts XXXVIII through L were prepared in the laboratory in accordance with the following general procedure:

The silica gel support, prewashed with hot oxalic acid solution to reduce impurities, was impregnated under vacuum with an aqueous solution of appropriate precursors denoted in Table A below. The solution volume was just sufficient to fill the catalyst pores, and the salt concentration was adjusted to give the desired weight percent in the finished catalyst. After standing 30 minutes at room temperature, the impregnated support was dried in stages, i.e., 85°C for 1 hour, 110°C for 2 hours, 150°C for 2 hours and 300°C for 2 hours. The dried material was transferred to a quartz tube, and reduced by heating to 500°C at a rate of 100°C per hour under flowing hydrogen. The temperature was held at 500°C for 1 hour and then the system was cooled to 80°C in hydrogen. The hydrogen was displaced with nitrogen and the catalyst cooled to room temperature.

TABLE A

| Identification | Catalyst Precursors |
|---|---|
| Catalyst XXXVIII | $Co(NO_3)_2$, $Ca(NO_3)_2$ |
| Catalyst XXXIX | $Ni(OAc)_2$ |
| Catalyst XL | $PdCl_2$, 6N HCl |
| Catalyst XLI | $PdCl_2$, $Ca(NO_3)_2$, 6N HCl |
| Catalyst XLII | $PdCl_2$, $Ca(NO_3)_2$, 6N HCl |
| Catalyst XLIII | $PdCl_2$, $Ca(NO_3)_2$, 6N HCl |
| Catalyst XLIV | $PdCl_2$, $Ca(NO_3)_2$, 6N HCl |
| Catalyst XLV | $PdCl_2$, $Ca(NO_3)_2$, 6N HCl |
| Catalyst XLVI | $PdCl_2$, $NH_4$ molybdate, 6N HCl |
| Catalyst XLVII | Chloroplatinic acid, $Ca(NO_3)_2$ |
| Catalyst XLVIII | $NH_4ReO_4$ |
| Catalyst XLIX | $RhCl_3$ |
| Catalyst L | $RhCl_3$, $Ca(NO_3)_2$ |

Catalysts LI through LIV were prepared in the laboratory in accordance with the following procedure:

The support, prewashed with hot oxalic acid in the case of silica gel, was impregnated under vacuum with a warm (70°C-80°C) toluene solution of the appropriate metal acetonyl acetonate complex listed in Table B below. The solution volume was just sufficient to fill the catalyt pores, and the weight of dissolved metal complex was adjusted to give the indicated weight percent metal in the finished catalyst. After standing 30 minutes at room temperature the solvent was evaporated in a nitrogen-purged oven by heating in stages, i.e., 85°C for 1 hour, 115°C for 2 hours, 150°C for 2 hours and 200°C for 2 hours. The dried impregnated support was transferred to a quartz tube and heated rapidly to 500°C in a stream of 50:50 ratio air and nitrogen. Heating at 500°C was continued for 3 hours under 100% air to complete oxidation of the

- 33 -

complex, then the system was flushed thoroughly with nitrogen. The nitrogen was replaced by a stream of 100% hydrogen introduced at 500°C and maintained for three hours. The catalyst was cooled to 80°C under hydrogen, then cooled to room temperature under a nitrogen purge.

## TABLE B

| Catalyst Identification | Catalyst Precursors |
|---|---|
| Catalyst LI | Fe Acetonyl Acetonate |
| Catalyst LII | Pd Acetonyl Acetonate |
| Catalyst LIII | Pt Acetonyl Acetonate |
| Catalyst LIV | Rh(CO)$_2$AcAc, Fe Acetonyl Acetate |

Catalysts LV through LVII were prepared in the laboratory in accordance with the following procedure:

The catalyst precursors listed in Table C below were dissolved in a minimum of hot water on a hot plate. The support was added with good mixing and most of the excess water was evaporated with frequent stirring and heating. The damp support was tranferred to a nitrogen-purged oven and dried in stages, i.e., 110°C for 2 hours and 150°C for 2 hours. The material was calcined in a porcelain dish by heating from 25°C to 500°C over a 3-hour period. After an additional 2 hours at 500°C the catalyst was removed and cooled to room temperature. The calcined catalyst was reduced in a quartz tube by heating to 400°C at at a rate of 100°C per hour under 100% hydrogen. After an additional 1 hour at 400°C the catalyst was cooled to 80°C in hydrogen. Final cooling to room temperature was carried out under nitrogen.

- 34 -

TABLE C

| Catalyst Identification | Catalyst Precursors |
|---|---|
| Catalyst LV | Ammonium paramolybdate |
| Catalyst LVI | Tantalum oxalate |
| Catalyst LVII | Ammonium paratungstate |

Catalyst LVIII was prepared in the laboratory in accordance with the following procedure:

A mixture of 2.88 grams (0.02 mole) of $MoO_3$, 1.591 grams (0.02 mole) of CuO and 0.480 grams (0.0048 mole) of $KNO_3$ was mixed by grinding to -200 mesh. The mixture was fired in a quartz boat in air at 800°C for 32 hours using a muffle furnace. The liquified product was cooled to room temperature, and the resultant solid ground to -200 mesh using a mortar and pestle.

## EXAMPLES 1 THROUGH 79

Examples 1 through 79 were conducted batchwise in a 300-milliliter stainless-steel autoclave reactor equipped with a magnetic stirrer in accordance with the following procedure. Prior to each of the examples the magnetic stirrer was disassembled and all components washed thoroughly to remove residual catalyst powder. All parts and internals of the reactor were similarly cleaned, rinsed with acetone and dried. The reactor was charged with a mixture of 50 grams of diethylene glycol and 1 gram of a finely pulverized catalyst specifically identified for each example in Table I below. (The reactor was charged with only 25 grams of diethylene glycol in Examples 18, 40, 47, 58 and 77 and with 100 grams of diethylene glycol in Examples 4, 15, 16, 19, 20, 21, 41, 46, 67, 72 and 73. The catalyst charge in Example 77 was only 0.5 grams.) The reactor was then sealed, purged twice with 500 psig of molecular hydrogen and, after pressure testing, charged with an initial amount of molecular hydrogen. The initial molecular hydrogen pressure for each example is given in Table I. The magnetic stirrer was started and adjusted to 1000 rpm, and the reaction system was then heated to a temperature specified for each example in Table I. After a six-hour reaction period the reactor was cooled, stirring was discontinued and the pressure vented slowly. (The reaction period was only two hours for Example 77, only four hours for Examples 8, 10, 12 and 29 and twenty hours for Example 75.) During the venting step, a sample of product gas was collected for analysis by gas chromatography. The remaining liquid contents of the reactor were then removed, weighed and analyzed by gas chromatography. The performance of catalysts tested in Examples 1 through 79 is given in Table I.

TABLE I

## POLYALKYLENE GLYCOL HYDROGENOLYSIS
### (BATCH PROCESS)
### PRODUCT DATA

| EXAMPLE | 1 | 2 | 3 | 4 | 5 | 6 | 7 | 8 | 9 |
|---|---|---|---|---|---|---|---|---|---|
| Catalyst Identification | I | II | III | III | III | IV | IV | IV | IV |
| Temperature, °C | 250 | 250 | 250 | 250 | 250 | 250 | 280 | 250 | 250 |
| Initial Pressure ($H_2$), psig bar | 2000 138 | 2000 138 | 600 41,4 | 1000 69 | 2000 138 | 250 17,2 | 500 34,5 | 500 34,5 | 1000 69 |
| **Reaction Rate (moles/kg/hr) to:** | | | | | | | | | |
| Ethylene Glycol | 3.43 | 3.41 | 3.90 | 1.97 | 6.50 | 3.72 | 4.83 | 6.59 | 4.88 |
| Ethanol | 4.15 | 4.34 | 4.75 | 2.51 | 8.48 | 4.69 | 6.27 | 7.66 | 7.01 |
| Ethane | 0.186 | 0.110 | 0.326 | ND | 0.80 | 0.158 | 0.204 | 0.150 | 0.212 |
| Monoethylene Glycol Monomethyl Ether | ND | ND | ND | .04 | ND | 0.15 | 1.07 | .242 | .187 |
| Monoethylene Glycol Monoethyl Ether | 0.081 | ND | 0.032 | 0.05 | 0.076 | 0.016 | 0.080 | 0.069 | 0.082 |
| Methanol | 0.321 | ND | 0.199 | 0.08 | 0.303 | 0.193 | 0.761 | 0.279 | 0.234 |
| Dioxane | 0.096 | ND | 0.155 | 0.05 | 0.103 | 0.656 | 1.42 | 0.238 | 0.091 |
| 2-Oxodioxane | ND | ND | 0.06 | ND | ND | 1.06 | 0.785 | 0.233 | 0.060 |
| 1,2-Butanediol[a] | ND | ND | 0.089 | 0.171 | 0.076 | 0.175 | 0.247 | 0.193 | 0.068 |
| Others[b] | 0.06 | ND | 0.017 | ND | 0.024 | 0.411 | 0.306 | 0.088 | 0.306 |
| **Selectivity (mole percent)** | | | | | | | | | |
| Ethylene Glycol | 83 | 87 | 81 | 77 | 79 | 56 | 51 | 80 | 72 |
| Ethylene Gycol & Ethanol | 91 | 99 | 89 | 88 | 91 | 63 | 59 | 87 | 88 |

[a] Small amounts of 4-butyrolactone and diethylene glycol monoethyl ether may also be included in this value.
[b] Includes n-butanol, acetaldehyde, diethyl ether, dimethyl ether, 2-methyldioxolane, 2,3-butanediol, propylene glycol, triethylene glycol and unknowns.

TABLE I (con't)

## POLYALKYLENE GLYCOL HYDROGENOLYSIS
### (BATCH PROCESS)
### PRODUCT DATA

| EXAMPLE | 10 | 11 | 12 | 13 | 14 | 15 | 16 | 17 | 18 |
|---|---|---|---|---|---|---|---|---|---|
| Catalyst Identification | IV | IV | IV | V | V | VI | VI | VI | V.I |
| Temperature, °C | 250 | 250 | 250 | 250 | 250 | 250 | 250 | 250 | 265 |
| Initial Pressure ($H_2$), psig bar | 1000  69 | 2000 138 | 2000 138 | 250 17,2 | 1000  69 | 500 34,5 | 1000  69 | 2000 138 | 2000 138 |
| **Reaction Rate (moles/kg/hr) to:** | | | | | | | | | |
| Ethylene Glycol | 7.22 | 11.89 | 10.48 | 2.15 | 2.38 | 1.37 | 1.62 | 3.18 | 2.57 |
| Ethanol | 8.60 | 26.05 | 18.70 | 3.35 | 8.21 | 1.23 | 1.81 | 3.18 | 2.79 |
| Ethane | 0.186 | 1.19 | 0.326 | 0.030 | 0.029 | 0.031 | 0.051 | 0.065 | 0.52 |
| Monoethylene Glycol Monomethyl Ether | 0.156 | 0.551 | 0.597 | 0.131 | .066 | ND | 0.10 | ND | ND |
| Monoethylene Glycol Monoethyl Ether | 0.082 | 0.484 | 0.316 | ND | 0.011 | ND | 0.03 | ND | 0.04 |
| Methanol | 0.267 | 1.82 | 2.84 | 0.083 | 0.078 | 0.060 | 0.106 | 0.15 | 4.35 |
| Dioxane | 0.104 | 0.345 | 0.531 | 0.236 | 0.047 | 0.13 | 0.05 | 0.13 | 0.11 |
| 2-Oxodioxane | 0.045 | 0.057 | 0.092 | 0.581 | 0.078 | 0.781 | 0.251 | ND | 0.032 |
| 1,2-Butanediol(a) | 0.158 | 0.697 | 0.503 | ND | ND | 0.133 | 0.132 | 0.074 | 0.148 |
| Others(b) | 0.093 | 1.71 | 0.705 | 0.072 | 0.089 | 0.136 | 0.308 | 0.039 | 0.208 |
| **Selectivity (mole percent)** | | | | | | | | | |
| Ethylene Glycol | 84 | 52 | 60 | 57 | 42 | 57 | 63 | 93 | 74 |
| Ethylene Gycol & Ethanol | 93 | 83 | 84 | 73 | 95 | 54 | 66 | 93 | 77 |

(a) Small amounts of 4-butyrolactone and diethylene glycol monoethyl ether may also be included in this value.
(b) Includes n-butanol, acetaldehyde, diethyl ether, dimethyl ether, 2-methyldioxolane, 2,3-butanediol, propylene glycol, triethylene glycol and unknowns.

TABLE I (con't)

## POLYALKYLENE GLYCOL HYDROGENOLYSIS
### (BATCH PROCESS)
### PRODUCT DATA

| EXAMPLE | 19 | 20 | 21 | 22 | 23 | 24 | 25 | 26 | 27 |
|---|---|---|---|---|---|---|---|---|---|
| Catalyst Identification | VI | VI | VI | VI | VII | VIII | VIII | IX | X |
| Temperature, °C | 275 | 275 | 275 | 250 | 250 | 250 | 250 | 250 | 250 |
| Initial Pressure ($H_2$), psig bar | 500 34,5 | 1000 69 | 2000 138 | 250 17,2 | 1000 69 | 1000 69 | 250 17,2 | 2000 138 | 1000 69 |
| **Reaction Rate (moles/kg/hr) to:** | | | | | | | | | |
| Ethylene Glycol | 4.25 | 5.17 | 7.86 | 2.10 | 2.63 | 5.61 | 0.577 | 8.28 | 2.45 |
| Ethanol | 2.29 | 4.38 | 7.86 | 0.99 | 2.65 | 6.53 | 0.777 | 9.24 | 3.37 |
| Ethane | 0.07 | 0.071 | 0.124 | 0.021 | 0.092 | 0.097 | 0.003 | 0.200 | 0.047 |
| Monoethylene Glycol Monomethyl Ether | 0.05 | 0.04 | 0.06 | 0.007 | 0.018 | 0.039 | ND | 0.078 | 0.125 |
| Monoethylene Glycol Monoethyl Ether | 0.02 | 0.02 | 0.04 | ND | 0.020 | 0.057 | ND | 0.053 | 0.027 |
| Methanol | 0.75 | 0.554 | 0.597 | 0.165 | 0.639 | 0.143 | 0.016 | 0.214 | 0.249 |
| Dioxane | 1.02 | 0.40 | 0.15 | 0.50 | 0.078 | 0.067 | 0.080 | ND | 0.165 |
| 2-Oxodioxane | 1.54 | 0.52 | 0.083 | 0.767 | 0.019 | 0.023 | 0.318 | ND | ND |
| 1,2-Butanediol[a] | 1.19 | 0.88 | 0.72 | 0.106 | 0.021 | 0.020 | ND | 0.086 | 0.033 |
| Others[b] | 1.13 | 0.977 | 0.996 | 0.370 | 0.082 | ND | 0.064 | 0.021 | 0.183 |
| **Selectivity (mole percent)** | | | | | | | | | |
| Ethylene Glycol | 52 | 68 | 79 | 63 | 90 | 89 | 51 | 91 | 71 |
| Ethylene Gycol & Ethanol | 40 | 63 | 79 | 47 | 90 | 97 | 59 | 96 | 85 |

[a] Small amounts of 4-butyrolactone and diethylene glycol monoethyl ether may also be included in this value.
[b] Includes n-butanol, acetaldehyde, diethyl ether, dimethyl ether, 2-methyldioxolane, 2,3-butanediol, propylene glycol, triethylene glycol and unknowns.

TABLE I (con't)

## POLYALKYLENE GLYCOL HYDROGENOLYSIS
### (BATCH PROCESS)
### PRODUCT DATA

| EXAMPLE | 28 | 29 | 30 | 31 | 32 | 33 | 34 | 35 | 36 |
|---|---|---|---|---|---|---|---|---|---|
| atalyst Identification | XI | XII | XIII | XIII | XIV | XIV | XV | XVI | XVII |
| Temperature, °C | 250 | 250 | 250 | 250 | 250 | 250 | 250 | 250 | 250 |
| Initial Pressure ($H_2$), psig bar | 1000  69 | 2000  138 | 1000  69 | 250  17,2 | 250  17,2 | 1000  69 | 1000  69 | 1000  69 | 250  17,2 |
| Reaction Rate (moles/kg/hr) to: | | | | | | | | | |
| Ethylene Glycol | 1.81 | 2.70 | 3.13 | 1.82 | 1.91 | 3.77 | 1.43 | 2.82 | 2.37 |
| Ethanol | 2.52 | 2.13 | 4.29 | 2.52 | 2.12 | 4.46 | 1.61 | 3.25 | 3.14 |
| Ethane | 0.60 | 0.239 | 0.157 | 0.068 | 0.061 | 0.091 | 0.593 | 0.886 | 0.056 |
| Monoethylene Glycol Monomethyl Ether | 0.453 | 0.108 | 0.142 | 0.059 | 0.026 | 0.041 | ND | 0.049 | 0.019 |
| Monoethylene Glycol Monoethyl Ether | 0.156 | 0.187 | 0.062 | ND | ND | 0.034 | ND | ND | 0.003 |
| Methanol | 0.40 | 0.161 | 0.190 | 0.154 | 0.067 | 0.110 | 0.440 | 0.085 | 0.051 |
| Dioxane | ND | 0.139 | 0.076 | 0.499 | 0.536 | 0.310 | ND | ND | 0.168 |
| 2-Oxodioxane | 0.035 | ND | 0.083 | 1.14 | 1.44 | 0.081 | ND | ND | 0.443 |
| 1,2-Butanediol[a] | ND | 0.073 | ND | ND | ND | ND | ND | ND | ND |
| Others[b] | 0.166 | 0.249 | ND | 0.259 | 0.229 | ND | 0.099 | ND | 0.270 |
| Selectivity (mole percent) | | | | | | | | | |
| Ethylene Glycol | 55 | 84 | 75 | 43 | 45 | 81 | 75 | 80 | 64 |
| Ethylene Gycol & Ethanol | 66 | 75 | 89 | 52 | 47 | 89 | 79 | 86 | 75 |

[a] Small amounts of 4-butyrolactone and diethylene glycol monoethyl ether may also be included in this value.
[b] Includes n-butanol, acetaldehyde, diethyl ether, dimethyl ether, 2-methyldioxolane, 2,3-butanediol, propylene glycol, triethylene glycol and unknowns.

TABLE I (cont')

## POLYALKYLENE GLYCOL HYDROGENOLYSIS
### (BATCH PROCESS)
### PRODUCT DATA

| EXAMPLE | 37 | 38 | 39 | 40 | 41 | 42 | 43 | 44 | 45 |
|---|---|---|---|---|---|---|---|---|---|
| Catalyst Identification | XVIII | XVIII | XXI | XIX | LVIII | XX | XXII | XXIII | XXIV |
| Temperature, °C | 250 | 250 | 250 | 250 | 250 | 250 | 250 | 250 | 250 |
| Initial Pressure ($H_2$), psig  bar | 1000  69 | 250  17,2 | 2000  138 | 1000  69 | 1000  69 | 2000  138 | 2000  138 | 2000  138 | 1000  69 |
| **Reaction Rate (moles/kg/hr) to:** | | | | | | | | | |
| Ethylene Glycol | 1.63 | 2.32 | 1.34 | 0.23 | 2.81 | 1.47 | 0.31 | 1.82 | 0.567 |
| Ethanol | 1.47 | 1.41 | 1.25 | 0.23 | 1.87 | 1.50 | 0.27 | 1.71 | 0.77 |
| Ethane | 0.079 | 0.027 | 0.019 | 0.133 | 1.72 | 0.019 | 0.006 | 0.024 | 0.013 |
| Monoethylene Glycol Monomethyl Ether | 0.07 | 0.009 | 0.04 | ND | ND | 0.06 | ND | ND | ND |
| Monoethylene Glycol Monoethyl Ether | ND | ND | 0.06 | 0.17 | 0.854 | 0.061 | 0.023 | 0.020 | ND |
| Methanol | 0.145 | 0.163 | 0.22 | ND | 0.27 | 0.22 | 0.28 | 0.14 | 0.066 |
| Dioxane | ND | 0.727 | 0.003 | 0.06 | 0.14 | ND | ND | 0.066 | 0.095 |
| 2-Oxodioxane | 0.034 | 1.27 | ND | ND | ND | ND | ND | 0.032 | 0.077 |
| 1,2-Butanediol(a) | ND | 0.205 | ND | ND | ND | ND | 0:048 | 079 | ND |
| Others(b) | 0.065 | 0.405 | 0.017 | ND | 0.043 | ND | 0.509 | 0.071 | ND |
| **Selectivity (mole percent)** | | | | | | | | | |
| Ethylene Glycol | 93 | 52 | 94 | 44 | 66 | 92 | 36 | 89 | 67 |
| Ethylene Gycol & Ethanol | 88 | 41 | 91 | 44 | 55 | 93 | 33 | 86 | 79 |

(a) Small amounts of 4-butyrolactone and diethylene glycol monoethyl ether may also be included in this value.

(b) Includes n-butanol, acetaldehyde, diethyl ether, dimethyl ether, 2-methyldioxolane, 2,3-butanediol, propylene glycol, triethylene glycol and unknowns.

TABLE I (con't)

## POLYALKYLENE GLYCOL HYDROGENOLYSIS
### (BATCH PROCESS)
### PRODUCT DATA

| EXAMPLE | 46 | 47 | 48 | 49 | 50 | 51 | 52 | 53 | 54 |
|---|---|---|---|---|---|---|---|---|---|
| Catalyst Identification | XXV | XXVI | XXXVIII | XXVII | XXVIII | XXIX | LI | XXX | XXXI |
| Temperature, °C | 250 | 250 | 250 | 250 | 250 | 250 | 250 | 250 | 250 |
| Initial Pressure (H$_2$), psig bar | 1000 69 | 2000 138 | 2000 138 | 2000 138 | 2000 138 | 2000 138 | 2000 138 | 2000 138 | 2000 138 |
| Reaction Rate (moles/kg/hr) to: | | | | | | | | | |
| Ethylene Glycol | 1.61 | 2.72 | 6.14 | 3.63 | 6.69 | 1.98 | 0.45 | 1.82 | 2.11 |
| Ethanol | 1.01 | 2.81 | 7.51 | 5.50 | 5.97 | 1.82 | 0.60 | 2.37 | 2.46 |
| Ethane | 0.341 | 0.15 | 1.89 | 0.274 | 0.605 | 0.046 | 0.023 | 0.129 | 0.10 |
| Monoethylene Glycol Monomethyl Ether | 0.63 | 1.34 | 1.85 | 1.34 | 0.57 | 0.19 | 0.016 | 0.27 | 0.29 |
| Monoethylene Glycol Monoethyl Ether | 0.34 | 0.446 | 3.36 | 0.628 | 2.02 | 0.33 | 0.037 | 0.61 | 0.71 |
| Methanol | 0.12 | 0.47 | 0.93 | ND | 4.60 | 1.57 | ND | 1.94 | 1.78 |
| Dioxane | 0.41 | ND | ND | 0.13 | 7.46 | 2.71 | ND | 2.79 | 2.10 |
| 2-Oxodioxane | 0.135 | 0.023 | ND | 0.06 | ND | 0.043 | 0.021 | 0.010 | 0.018 |
| 1,2-Butanediol(a) | ND | ND | ND | ND | ND | ND | 0.055 | ND | ND |
| Others(b) | 0.082 | ND | 0.036 | 0.64 | 2.19 | 0.241 | 0.242 | 0.377 | 0.28 |
| Selectivity (mole percent) | | | | | | | | | |
| Ethylene Glycol | 52 | 59 | 47 | 49 | 35 | 36 | 50 | 29 | 37 |
| Ethylene Gycol & Ethanol | 43 | 59 | 52 | 62 | 34 | 35 | 58 | 34 | 40 |

(a) Small amounts of 4-butyrolactone and diethylene glycol monoethyl ether may also be included in this value.
(b) Includes n-butanol, acetaldehyde, diethyl ether, dimethyl ether, 2-methyldioxolane, 2,3-butanediol, propylene glycol, triethylene glycol and unknowns.

- 42 -

TABLE I (con't)

## POLYALKYLENE GLYCOL HYDROGENOLYSIS
### (BATCH PROCESS)
### PRODUCT DATA

| EXAMPLE | 55 | 56 | 57 | 58 | 59 | 60 | 61 | 62 | 63 |
|---|---|---|---|---|---|---|---|---|---|
| Catalyst Identification | XXXII | LV | XXXIX | XXXIII | LII | XL | XLI | XLII | XLIII |
| Temperature, °C | 250 | 250 | 250 | 250 | 250 | .250 | 250 | 250 | 250 |
| Initial Pressure ($H_2$), psig bar | 2000 138 | 2000 138 | 2000 138 | 1000 69 | 2000 138 | 2000 138 | 2000 138 | 2000 138 | 2000 138 |
| **Reaction Rate (moles/kg/hr) to:** | | | | | | | | | |
| Ethylene Glycol | 2.08 | 3.81 | 11.62 | 0.18 | 1.48 | 2.20 | 1.31 | 1.10 | 0.99 |
| Ethanol | 1.68 | 3.51 | 13.71 | 0.10 | 0.98 | 1.96 | 1.34 | 0.93 | 1.12 |
| Ethane | 0.34 | 0.11 | 0.204 | 0.063 | 0.023 | 0.072 | 0.017 | 0.031 | 0.021 |
| Monoethylene Glycol Monomethyl Ether | 0.24 | 0.673 | 3.83 | 0.41 | 0.092 | 0.415 | 0.671 | 0.382 | 0.448 |
| Monoethylene Glycol Monoethyl Ether | 0.70 | 0.172 | 0.344 | 0.09 | 0.143 | 0.210 | 0.188 | 0.161 | 0.122 |
| Methanol | 1.30 | 0.298 | 1.23 | 0.07 | 0.462 | 0.297 | 0.61 | 0.327 | 0.342 |
| Dioxane | 1.15 | 0.798 | 0.791 | 0.02 | 0.043 | 0.049 | ND | 0.108 | ND |
| 2-Oxodioxane | 0.05 | 0.028 | 0.007 | ND | ND | 0.01 | 0.079 | ND | 0.086 |
| 1,2-Butanediol[a] | ND | 0.041 | ND | ND | ND | 0.014 | ND | 0.043 | ND |
| Others[b] | 0.157 | 0.914 | 0.093 | 0.072 | 0.33 | 1.13 | 0.012 | 0.230 | 0.033 |
| **Selectivity (mole percent)** | | | | | | | | | |
| Ethylene Glycol | 48 | 60 | 65 | 24 | 80 | 56 | 57 | 56 | 56 |
| Ethylene Gycol & Ethanol | 43 | 58 | 71 | 18 | 66 | 53 | 58 | 52 | 60 |

[a] Small amounts of 4-butyrolactone and diethylene glycol monoethyl ether may also be included in this value.
[b] Includes n-butanol, acetaldehyde, diethyl ether, dimethyl ether, 2-methyldioxolane, 2,3-butanediol, propylene glycol, triethylene glycol and unknowns.

## TABLE I (con't)

## POLYALKYLENE GLYCOL HYDROGENOLYSIS
## (BATCH PROCESS)
## PRODUCT DATA

| EXAMPLE | 64 | 65 | 66 | 67 | 68 | 69 | 70 | 71 | 72 |
|---|---|---|---|---|---|---|---|---|---|
| Catalyst Identification | XLIV | XLV | XLVI | XXXIV | XXXV | LIII | XLVII | XLVIII | XXXVI |
| Temperature, °C | 250 | 250 | 250 | 250 | 250 | 250 | 250 | 250 | 250 |
| Initial Pressure (H₂), psig/bar | 2000/138 | 2000/138 | 2000/138 | 1000/69 | 2000/138 | 2000/138 | 2000/138 | 2000/138 | 1000/69 |
| **Reaction Rate (moles/kg/hr) to:** | | | | | | | | | |
| Ethylene Glycol | 1.22 | 1.71 | 10.54 | 0.88 | 1.56 | 5.97 | 3.04 | 3.52 | 0.36 |
| Ethanol | 1.39 | 1.92 | 25.27 | 0.32 | 0.66 | 4.33 | 2.71 | 4.76 | 0.19 |
| Ethane | 0.055 | 0.017 | 2.67 | 0.10 | 0.279 | 0.401 | 0.268 | 4.44 | 0.095 |
| Monoethylene Glycol Monomethyl Ether | 0.334 | 0.539 | 0.316 | 0.09 | ND | 0.167 | 0.114 | 0.172 | 0.12 |
| Monoethylene Glycol Monoethyl Ether | 0.165 | 0.174 | 3.39 | 0.29 | 0.081 | 2.82 | 0.479 | 2.20 | 0.04 |
| Methanol | 0.259 | 0.521 | 1.25 | 0.07 | ND | 0.256 | 0.223 | 0.66 | 0.14 |
| Dioxane | 0.114 | 0.038 | 0.098 | 3.31 | 1.33 | 0.064 | ND | 0.105 | 0.61 |
| 2-Oxodioxane | ND | 0.007 | 0.027 | ND | 0.013 | ND | 0.022 | 0.019 | 0.182 |
| 1,2-Butanediol[a] | 0.278 | ND | 0.015 | ND | 0.968 | ND | 0.018 | ND | ND |
| Others[b] | 0.346 | 0.248 | 0.166 | 0.091 | 0.086 | 0.143 | 0.401 | 0.197 | ND |
| **Selectivity (mole percent)** | | | | | | | | | |
| Ethylene Glycol | 47 | 60 | 45 | 20 | 42 | 70 | 75 | 39 | 28 |
| Ethylene Gycol & Ethanol | 51 | 64 | 77 | 14 | 30 | 60 | 71 | 46 | 22 |

[a] Small amounts of 4-butyrolactone and diethylene glycol monoethyl ether may also be included in this value.
[b] Includes n-butanol, acetaldehyde, diethyl ether, dimethyl ether, 2-methyldioxolane, 2,3-butanediol, propylene glycol, triethylene glycol and unknowns.

TABLE I (con't)

## POLYALKYLENE GLYCOL HYDROGENOLYSIS
### (BATCH PROCESS)
### PRODUCT DATA

| EXAMPLE | 73 | 74 | 75 | 76 | 77 | 78 | 79 |
|---|---|---|---|---|---|---|---|
| Catalyst Identification | XLIX | XLIX | LIV | L | XXXVII | LVI | LVII |
| Temperature, °C | 250 | 250 | 250 | 250 | 250 | 250 | 250 |
| Initial Pressure (H₂), psig bar | 1000 69 | 2000 138 | 2000 138 | 2000 138 | 1000 69 | 2000 138 | 2000 138 |
| **Reaction Rate (moles/kg/hr) to:** | | | | | | | |
| Ethylene Glycol | 3.13 | 9.86 | 1.18 | 13.22 | 33.4 | 0.714 | 1.17 |
| Ethanol | 0.66 | 5.54 | 1.54 | 11.48 | 2.01 | 0.96 | 0.86 |
| Ethane | 1.66 | 11.82 | 0.045 | 5.75 | 5.98 | 0.111 | 0.008 |
| Monoethylene Glycol Monomethyl Ether | 1.57 | 8.70 | 1.47 | 0.791 | 47.1 | 0.081 | 0.18 |
| Monoethylene Glycol Monoethyl Ether | 0.98 | 4.82 | 0.29 | 2.42 | 2.53 | 0.111 | 0.076 |
| Methanol | 0.34 | 1.94 | 1.23 | 0.745 | 5.19 | 0.305 | 0.653 |
| Dioxane | 4.19 | 2.15 | 0.016 | 0.136 | 0.16 | 0.062 | 0.041 |
| 2-Oxodioxane | 0.03 | ND | ND | ND | 0.255 | 0.04 | ND |
| 1,2-Butanediol(a) | ND | ND | ND | ND | ND | ND | ND |
| Others(b) | ND | 0.398 | 0.01 | 0.181 | 0.111 | 0.493 | 0.079 |
| **Selectivity (mole percent)** | | | | | | | |
| Ethylene Glycol | 33 | 33 | 37 | 70 | 47 | 43 | 84 |
| Ethylene Gycol & Ethanol | 20 | 26 | 43 | 66 | 25 | 50 | 73 |

(a)Small amounts of 4-butyrolactone and diethylene glycol monoethyl ether may also be included in this value.
(b)Includes n-butanol, acetaldehyde, diethyl ether, dimethyl ether, 2-methyldioxolane, 2,3-butanediol, propylene glycol, triethylene glycol and unknowns.

- 46 -

Examples 1 through 39 illustrate the overall effectiveness of commercially available copper chromite catalysts as the hydrogenation catalyst of the present invention. Copper chromite is the most preferred hydrogenation catalyst for use in the hydrogenolysis process of this invention. When copper chromite is used as the hydrogenation catalyst, the selectivities of monoethylene glycol and ethanol may approach 95 molar percent each (see Examples 17, 23, 26 and 39). Example 10 shows that the combined rate of monoethylene glycol and ethanol formation from diethylene glycol was greater than 15 moles/kg cat./hr. or 70 lbs./cu. ft./hr. This rate is sufficiently high to be commercially desirable. Copper chromite has the further advantage of being relatively inexpensive and commercially available from a number of suppliers in a variety of formulations.

Examples 15, 16 and 17 illustrate the effect of pressure on the hydrogenolysis reaction using the same hydrogenation catalyst, i.e., copper chromite (Catalyst VI), and the same temperature. As can be seen from Example 17, a pressure of about 2000 psig is preferred for obtaining overall desirable performance. It is noted that lower pressures tend to decrease the production rate and selectivity of ethanol. (See also Examples 19, 20 and 21.) The most preferred pressure for monoethylene glycol and ethanol production ranges from about 1000 psig to about 3000 psig.

Examples 17, 18 and 21 illustrate the effect of temperature on the hydrogenolysis reaction using the same hydrogenation catalyst, i.e., copper chromite (Catalyst VI), and the same pressure. As can be seen from Example 17, a temperature of about 250°C is preferred for obtaining overall desirable performance. The most preferred temperature for monoethylene glycol and ethanol production ranges from about 225°C to about 265°C.

Examples 40 through 79 illustrate the effectiveness of other hydrogenation catalysts utilized in the hydrogenolysis process of this invention.

Substantial promotional effects of calcium and molybdenum additives were observed on both the rate and selectivity of some catalysts. A comparison of Examples 74 and 76 illustrates a marked rate enhancement of monoethylene glycol and ethanol formation from the addition of 2 percent by weight calcium (as the oxide) to a 2.5 percent by weight rhodium catalyst. Also, from a comparison of Examples 74 and 76, it can be seen that the selectivity of monoethylene glycol-ethanol production is increased from about 26 molar percent to about 66 molar percent. A comparison of Examples 60 and 66 shows that the addition of 2 percent by weight molybdenum to a 5 percent by weight palladium catalyst served to enhance the rate of monoethylene glycol and ethanol formation almost ten-fold. This rate enhancement was accompanied by an increase in monoethylene glycol-ethanol selectivity of from about 53 molar percent to about 77 molar percent.

## EXAMPLES 80 THROUGH 110

Examples 80 through 110 were conducted under continuous conditions in a 3/4 inch outside diameter by 16-inch stainless-steel tube reactor (35-milliliters volume) which was coaxially fitted

– 48 –

with a 1.5-millimeter diameter stainless-steel-sheathed thermocouple in accordance with the following procedure. A 10-gram charge of catalyst, specifically identified for each example in Table II below, was dispersed in an equal volume of glass helices and placed in the stainless steel tube reactor with beds of glass helices occupying the space above and below the charged catalyst. The catalyst was then reduced in the stainless steel tube reactor by heating to 150°C under flowing nitrogen. Molecular hydrogen was introduced and adjusted to a concentration of about 10 volume percent. The temperature and the molecular hydrogen concentration were then increased incrementally over a 48-hour period until the temperature reached 265°C and the reducing atmosphere was 100 volume percent molecular hydrogen. After reduction, the temperature and molecular hydrogen flow rate were adjusted to their desired levels. Diethylene glycol and molecular hydrogen were premixed at 275°C in a preheater filled with glass helices and of dimensions identical to that of the stainless steel tube reactor. The reactants were then passed downward over the catalyst bed at conditions of temperature, pressure, gas and liquid flow rates (gas hourly space velocity and liquid hourly space velocity) as indicated in Table II. The products were condensed and collected at reactor pressure. The liquid products and uncondensed gases were sampled periodically and analyzed by gas chromatography. Uncoverted molecular hydrogen was not recycled. The performance of catalysts tested in Examples 80 through 110 is given in Table II.

| EXAMPLE | 80 | 81 | 82 | 83 | 84 | 85 | 86 | 87 | 88 |
|---|---|---|---|---|---|---|---|---|---|
| Catalyst Identification | VI | VI | VI | VI | VI | VI | VI | VI | VI |
| Temperature, °C | 250 | 250. | 250 | 250 | 265 | 225 | 225 | 225 | 250 |
| Pressure ($H_2$), psig  bar | 500 34,5 | 1000 69 | 2000 138 | 3000 207 | 1000 69 | 1000 69 | 1000 69 | 1000 69 | 1000 69 |
| Gas Hourly Space Velocity ($H_2$), hr$^{-1}$ | 23,000 | 23,000 | 44,000 | 26,000 | 24,000 | 22,000 | 23,000 | 20,000 | 24,000 |
| Liquid Hourly Space Velocity (DEG), hr$^{-1}$ | 1.2 | 1.2 | 1.1 | 1.1 | 1.2 | 4.1 | 2.0 | 1.1 | 4.1 |
| **Reaction Rate (moles/kg/hr) to:** | | | | | | | | | |
| Ethylene Glycol | 1.46 | 1.97 | 2.67 | 2.31 | 2.30 | 1.92 | 1.90 | 1.63 | 5.11 |
| Ethanol | 1.86 | 2.71 | 3.81 | 3.66 | 3.58 | 1.94 | 2.06 | 1.86 | 6.08 |
| Ethane | 0.031 | 0.032 | 0.034 | 0.040 | 0.048 | 0.014 | 0.014 | 0.012 | 0.052 |
| Monoethylene Glycol Monomethyl Ether | 0.016 | 0.009 | 0.005 | 0.006 | 0.015 | ND | 0.001 | ND | 0.005 |
| Monoethylene Glycol Monoethyl Ether | 0.002 | 0.002 | 0.002 | 0.003 | 0.005 | ND | ND | 0.001 | 0.005 |
| Methanol | 0.055 | 0.051 | 0.046 | 0.061 | 0.110 | 0.036 | 0.022 | 0.405 | 0.127 |
| Dioxane | ND | ND | ND | ND | ND | ND | ND | ND | ND |
| 2-Oxodioxane | 0.055 | 0.012 | 0.002 | ND | 0.014 | 0.017 | 0.007 | 0.007 | 0.043 |
| 1,2-Butanediol[a] | 0.057 | 0.066 | 0.078 | 0.123 | 0.151 | 0.006 | 0.023 | 0.030 | 0.177 |
| Others[b] | 0.086 | 0.086 | 0.091 | 0.160 | 0.216 | 0.015 | 0.046 | 0.004 | 0.215 |
| **Selectivity (mole percent)** | | | | | | | | | |
| Ethylene Glycol | 78 | 78 | 79 | 70 | 69 | 97 | 94 | 89 | 85 |
| Ethylene Gycol & Ethanol | 89 | 92 | 96 | 91 | 88 | 98 | 98 | 95 | 93 |

[a] Small amounts of 4-butyrolactone and diethylene glycol monoethyl ether may also be included in this value.
[b] Includes n-butanol, acetaldehyde, diethyl ether, dimethyl ether, 2-methyldioxolane, 2,3-butanediol, propylene glycol, triethylene glycol and unknowns.

| EXAMPLE | 89 | 90 | 91 | 92 | 93 | 94 | 95 | 96 | 97 |
|---|---|---|---|---|---|---|---|---|---|
| Catalyst Identification | VI | VI | V | V | V | VII | VII | VII | VII |
| Temperature, °C | 250 | 265 | 250 | 250 | 265 | 250 | 250 | 250 | 250 |
| Pressure (H$_2$), psig  bar | 1000  69 | 1000  69 | 250  17,2 | 1000 69 | 1000  69 | 250  17,2 | 1000  69 | 1000 69 | 2000 138 |
| Gas Hourly Space Velocity (H$_2$), hr$^{-1}$ | 24,000 | 23,000 | 22,000 | 23,000 | 24,000 | 23,000 | 22,000 | 23,000 | 21,000 |
| Liquid Hourly Space Velocity (DEG), hr$^{-1}$ | 1.1 | 1.1 | 1.2 | 1.2 | 1.1 | 1.1 | 1.1 | 0.4 | 1.1 |
| Reaction Rate (moles/kg/hr) to: | | | | | | | | | |
| Ethylene Glycol | 3.07 | 3.73 | 1.40 | 1.52 | 2.27 | 1.12 | 1.75 | 1.40 | 2.49 |
| Ethanol | 4.29 | 5.97 | 1.54 | 2.07 | 3.74 | 1.57 | 2.57 | 2.07 | 3.34 |
| Ethane | 0.037 | 0.055 | 0.032 | 0.110 | 0.208 | 0.036 | 0.029 | 0.027 | 0.035 |
| Monoethylene Glycol Monomethyl Ether | 0.007 | 0.011 | 0.042 | 0.018 | 0.053 | 0.011 | 0.006 | 0.004 | 0.003 |
| Monoethylene Glycol Monoethyl Ether | 0.004 | 0.006 | 0.015 | 0.015 | 0.033 | 0.006 | 0.005 | 0.003 | 0.003 |
| Methanol | 0.091 | 0.196 | 0.084 | 0.117 | 0.192 | 0.071 | 0.058 | 0.039 | 0.069 |
| Dioxane | ND | ND | 0.023 | 0.013 | 0.023 | ND | ND | ND | 0.007 |
| 2-Oxodioxane | 0.007 | 0.006 | 0.207 | 0.009 | 0.012 | 0.228 | 0.009 | 0.007 | 0.002 |
| 1,2-Butanediol(a) | 0.130 | 0.273 | 0.132 | 0.110 | 0.158 | 0.081 | 0.080 | 0.054 | 0.098 |
| Others(b) | 0.157 | 0.328 | 0.218 | 0.108 | 0.259 | 0.154 | 0.120 | 0.068 | 0.155 |
| Selectivity (mole percent) | | | | | | | | | |
| Ethylene Glycol | 77 | 68 | 67 | 73 | 63 | 67 | 74 | 75 | 78 |
| Ethylene Gycol & Ethanol | 92 | 89 | 70 | 86 | 83 | 75 | 91 | 93 | 92 |

(a)Small amounts of 4-butyrolactone and diethylene glycol monoethyl ether may also be included in this value.
(b)Includes n-butanol, acetaldehyde, diethyl ether, dimethyl ether, 2-methyldioxolane, 2,3-butanediol, propylene glycol, triethylene glycol and unknowns.

| EXAMPLE | 98 | 99 | 100 | 101 | 102 | 103 | 104 | 105 | 106 |
|---|---|---|---|---|---|---|---|---|---|
| Catalyst Identification | IV | IV | IV | IV | IV | IV | IV | IV | IV |
| Temperature, °C | 225 | 225 | 250 | 250 | 250 | 250 | 250 | 250 | 250 |
| Pressure ($H_2$), psig bar | 1000 69 | 1000 69 | 1000 69 | 1000 69 | 1000 69 | 2000 138 | 2000 138 | 1000 69 | 2000 138 |
| Gas Hourly Space Velocity ($H_2$), hr$^{-1}$ | 20,000 | 40,000 | 21,000 | 40,000 | 40,000 | 53,000 | 57,000 | 20,000 | 20,000 |
| Liquid Hourly Space Velocity (DEG), hr$^{-1}$ | 0.53 | 0.53 | 1.1 | 0.53 | 1.1 | 0.53 | 1.1 | 1.1 | 1.1 |
| **Reaction Rate (moles/kg/hr) to:** | | | | | | | | | |
| Ethylene Glycol | 0.56 | 0.67 | 1.97 | 1.53 | 2.48 | 1.35 | 2.30 | 2.09 | 2.22 |
| Ethanol | 0.65 | 0.79 | 3.29 | 2.54 | 3.51 | 2.33 | 3.46 | 3.16 | 3.35 |
| Ethane | 0.047 | ND | 0.066 | 0.043 | 0.103 | 0.068 | 0.073 | 0.053 | 0.069 |
| Monoethylene Glycol Monomethyl Ether | 0.048 | 0.062 | 0.202 | 0.158 | 0.151 | 1.29 | 0.106 | 0.313 | 0.372 |
| Monoethylene Glycol Monoethyl Ether | ND | ND | 0.023 | 0.016 | 0.013 | 0.016 | 0.017 | 0.011 | 0.016 |
| Methanol | 0.020 | 0.028 | 0.129 | 0.135 | 0.125 | 0.111 | 0.077 | 0.112 | 0.167 |
| Dioxane | ND | ND | 0.025 | 0.012 | 0.018 | 0.014 | 0.018 | 0.007 | 0.009 |
| 2-Oxodioxane | ND | ND | 0.007 | 0.002 | 0.006 | ND | ND | 0.012 | ND |
| 1,2-Butanediol[a] | 0.012 | 0.015 | 0.127 | 0.080 | 0.118 | 0.050 | 0.079 | 0.088 | 0.025 |
| Others[b] | 0.010 | 0.012 | 0.119 | 0.093 | 0.114 | 0.053 | 0.059 | 0.108 | 0.023 |
| **Selectivity (mole percent)** | | | | | | | | | |
| Ethylene Glycol | 84 | 83 | 67 | 63 | 72 | 64 | 73 | 69 | 68 |
| Ethylene Gycol & Ethanol | 91 | 90 | 84 | 84 | 88 | 88 | 91 | 83 | 86 |

[a] Small amounts of 4-butyrolactone and diethylene glycol monoethyl ether may also be included in this value.
[b] Includes n-butanol, acetaldehyde, diethyl ether, dimethyl ether, 2-methyldioxolane, 2,3-butanediol, propylene glycol, triethylene glycol and unknowns.

TABLE II (cont')
POLYALKYLENE GLYCOL HYDROGENOLYSIS
(CONTINUOUS PROCESS)
PRODUCT DATA

| EXAMPLE | 107 | 108 | 109 | 110 |
|---|---|---|---|---|
| Catalyst Identification | IV | IV | IV | IV |
| Temperature, °C | 250 | 250 | 250 | 265 |
| Pressure ($H_2$), psig bar | 2000 138 | 2000 138 | 3000 207 | 3000 207 |
| Gas Hourly Space Velocity ($H_2$), hr$^{-1}$ | 20,000 | 20,000 | 20,000 | 20,000 |
| Liquid Hourly Space Velocity (DEG), hr$^{-1}$ | 2.0 | 4.0 | 2.0 | 2.0 |
| **Reaction Rate (moles/kg/hr) to:** | | | | |
| Ethylene Glycol | 2.47 | 2.41 | 1.89 | 2.65 |
| Ethanol | 3.19 | 3.02 | 2.79 | 4.53 |
| Ethane | 0.059 | 0.072 | 0.066 | 0.102 |
| Monoethylene Glycol Monomethyl Ether | 0.288 | 0.252 | 0.212 | 0.300 |
| Monoethylene Glycol Monoethyl Ether | 0.012 | 0.010 | 0.013 | 0.017 |
| Methanol | 0.138 | 0.099 | 0.100 | 0.191 |
| Dioxane | 0.003 | ND | 0.009 | 0.016 |
| 2-Oxodioxane | ND | .009 | ND | ND |
| 1,2-Butanediol[a] | 0.029 | 0.035 | 0.031 | 0.069 |
| Others[b] | 0.043 | 0.053 | 0.066 | 0.091 |
| **Selectivity (mole percent)** | | | | |
| Ethylene Glycol | 75 | 78 | 72 | 65 |
| Ethylene Gycol & Ethanol | 88 | 88 | 89 | 88 |
| Others[c] | 12 | 12 | 11 | 12 |
| Ethylene Gycol/Ethanol[d] | 0.78 | 0.80 | 0.68 | 0.59 |

[a] Small amounts of 4-butyrolactone and diethylene glycol monoethyl ether may also be included in this value.

[b] Includes n-butanol, acetaldehyde, diethyl ether, dimethyl ether, 2-methyldioxolane, 2,3-butanediol, propylene glycol, triethylene glycol and unknowns.

- 53 -

Examples 80 through 110 illustrate the overall effectiveness of the present invention when employing a continuous process. Copper chromite is the most preferred hydrogenation catalyst for use in the continuous process of this invention. The selectivities of monoethylene glycol and ethanol may approach or exceed 95 molar percent (see Examples 85 and 86) when copper chromite is used as the hydrogenation catalyst in the continuous process. The combined rate of monoethylene glycol and ethanol formation resulting from the continuous process is also sufficiently high to be commercially desirable (see Examples 88, 89 and 90). Examples 80 through 110 illustrate the wide temperature range and pressure range which may be employed in the continuous process. Temperatures ranging from about 225°C to about 265°C (see Examples 85, 90, 98 and 110) and pressures ranging from about 500 psig to about 3,000 psig (see Examples 80, 83 and 109) are most desirable for obtaining preferred monoethylene glycol-ethanol selectivities and rates. The gas hourly space velocity (GHSV) and liquid hourly space velocity (LHSV) may also be varied over a wide range utilizing the continuous process. Gas hourly space velocities of from about 20,000 $hr^{-1}$ to about 57,000 $hr^{-1}$ (see Examples 98 and 104) and liquid hourly space velocities of from about 0.4 $hr^{-1}$ to about 4.1 $hr^{-1}$ (see Examples 88 and 96) are highly desirable for obtaining preferred monoethylene glycol-ethanol selectivites and rates. The most preferred GHSV can range from about 5,000 $hr^{-1}$ to about 75,000 $hr^{-1}$ and the most preferred LHSV can range from about 0.25 $hr^{-1}$ to about 10 $hr^{-1}$.

PATENTANWÄLTE

WUESTHOFF - v. PECHMANN - BEHRENS - GOETZ

EUROPEAN PATENT ATTORNEYS

DR.-ING. FRANZ WUESTHOFF
DR. PHIL. FREDA WUESTHOFF (1927-1956)
DIPL-ING. GERHARD PULS (1952-1971)
DIPL-CHEM. DR. E. FREIHERR VON PECHMANN
DR.-ING. DIETER BEHRENS
DIPL-ING.; DIPL.-WIRTSCH.-ING. RUPERT GOETZ

D-8000 MÜNCHEN 90
SCHWEIGERSTRASSE 2

TELEFON: (089) 66 20 51
TELEGRAMM: PROTECTPATENT
TELEX: 524 070

EP-56 537

13231

— 54 —

Claims


1. A process for cleaving a polyalkylene glycol preferably diethylene glycol containing at least one ether group therein at a carbon - to-oxygen covalent bond which comprises heating such a polyalkylene glycol with molecular hydrogen in the presence of a hydrogenation catalyst to produce at least one of monoethylene glycol and ethanol.


2. The process of claim 1 wherein the hydrogenation catalyst is a composition comprising at least one of copper, chromium, molybdenum, tungsten, iron, cobalt, nickel, ruthenium, rhodium, palladium, platinum, iridium, manganese, rhenium, tantalum, vanadium, niobium or zinc.


3. The process of claim 1 wherein the hydrogenation catalyst is a composition comprising copper and chromium or copper, chromium and barium or copper, chromium and manganese or rhodium and calcium or palladium and molybdenum.

4. The process of claim 1 or 2 wherein the polyalkylene glycol and molecular hydrogen are present in a molar ratio of molecular hydrogen to polyalkylene glycol of from about 1:20 to about 2000:1.

5. The process of claim 1 to 3 wherein the hydrogenation catalyst is present in an amount of from about 0.01 weight percent to about 50 weight percent based on the total weight of the reaction mixture.

6. The process of claim 1 to 4 wherein the polyalkylene glycol has a liquid hourly space velocity of from about 0.01 $hr^{-1}$ to about 100 $hr^{-1}$ and molecular hydrogen has a gas hourly space velocity of from about 100 $hr^{-1}$ to about 100,000 $hr^{-1}$.

7. The process of claim 1 to 5 wherein the reaction temperature is from about 150°C to about 350°C.

8. The process of claim 1 wherein the initial pressure resulting from molecular hydrogen is from about 500 psig to about 4000 psig.

9. The process of claim 1 wherein the monethylene glycol and ethanol are produced at a selectivity of at least about 50 molar percent.

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | DE-C-  417 926  (E.MERCK) <br> *The whole document* | 1-9 | C 07 C  29/10 <br> C 07 C  29/60 <br> C 07 C  31/20 <br> C 07 C  31/08 |
| A | DE-C-  898 894  (B.A.S.F.) <br> *The whole document* | 1-9 | |
| A | EP-A-0 030 397  (SHELL) | 1 | |
| A | US-A-3 428 692  (C.M.STARKS et al.) <br> *Example 2* | 1 | |
| A | DE-A-1 793 182  (BAYER) <br> *Claims* | 1 | |
| D,A | CHEMICAL ABSTRACTS, vol. 83, no. 1, 7th July 1975, page 762, no. 9186m, Columbus Ohio (USA); <br> & JP - A - 74 25 246 (MITSUI TOATSU CHEMICALS, INC.) (28-06-1974) *Abstract* | 1 | **TECHNICAL FIELDS SEARCHED (Int. Cl. ³)** <br><br> C 07 C  29/00 <br> C 07 C  43/00 <br> C 07 C  41/00 |

The present search report has been drawn up for all claims

| Place of search <br> THE HAGUE | Date of completion of the search <br> 06-01-1983 | Examiner <br> DELHOMME H.A. |
|---|---|---|

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1503. 03.82